# EUROPEAN PATENT SPECIFICATION

(11) **EP 0 437 523 B1**
(45) Date of publication and mention of the grant of the patent: **08.09.1993**
(21) Application number: 89911784.0
(22) Date of filing: 10.10.1989
(51) Int. Cl.: C11C 3/08, A61K 31/23

(54) **TRIGLYCERIDES, NUTRITIONAL COMPOSITION COMPRISING SUCH TRIGLYCERIDES, AND USE OF THE NUTRITIONAL COMPOSITION FOR NUTRITION**
TRIGLYCERIDE, NAHRUNGSZUSAMMENSETZUNG DARAUS UND DEREN VERWENDUNG ALS NAEHRMITTEL
TRIGLYCERIDES, COMPOSITION NUTRITIONNELLE LES COMPRENANT, ET UTILISATION DE LA COMPOSITION NUTRITIONNELLE POUR LA NUTRITION

(30) Priority: 10.10.1988 DK 5652/88
(43) Date of publication of application: 24.07.1991
(73) Proprietor: NOVO NORDISK A/S, 2880 Bagsvaerd (DK)
(72) Inventor: HANSEN, Tomas, Tage, DK-3450 Alleroed (DK); GODTFREDSEN, Sven, Erik, DK-3500 Vaerloese (DK)
(74) Representative: Bach, Niels
(86) International application number: DK8900233
(87) International publication number: WO9004009

(56) References cited:
- US-A- 4 753 963

## Description

The invention relates to triglycerides, a nutritional composition comprising such triglycerides, and a use of the nutritional composition for nutrition.

Soy bean oil is an extremely important nutritional material which is used extensively for enteral as well as for parenteral nutrition. The oil is composed of triglycerides i.e. glycerol molecules esterified with a variety of fatty acids characteristic for the soy bean oil. More than 95% of these fatty acids are the unbranched saturated and unsaturated fatty acids denoted C_{16.0}, C_{18.0}, C_{18.1}, C_{18.2}, C_{18.3}, the first subscript indicating the number of carbon atoms, and the second subscript indicating the number of double bonds. In the present specification with claims "soy" will signify the corresponding mixture of acid residues.

Even though soy bean oil plays an important role as a nutrient, triglycerides containing long chain fatty acids only (such as those in soy oil) are, in fact, known to be absorbed more slowly than the corresponding triglycerides containing medium chain length acids only (C₈-C₁₀) (Metabolism, Vol. 38, p. 507-513, 1989). It is also known that the human lipases, especially human pancreatic lipase, have a low activity towards long chain and polyunsaturated acids as those occurring in soy oil (Lipids, Vol. 22, 711-714, 1987). This is the case also for the lipases as e.g. the lipoprotein lipase involved in cleavage of triglycerides applied as emulsions in parenteral nutrition. In spite of its extensive use in parenteral and enteral nutrition the bioavailability of soy oil is thus not optimal and needs improvement. This is particularly so in connection with nutrition of elderly or diseased patients in whom the enzymes involved in lipid metabolism are not available in sufficient quantities to ensure an optimal utilization of soy bean oil containing nutritional conpositions.

The purpose of the present invention is thus provision of triglycerides which are devoid of the problems mentioned above and which can thus be formulated as an enterally administrable composition, which when used as a nutrient exhibits an improved bioavailability in regard to soy acids, or which can be formulated as a parenterally administrable composition, which exhibits an improved bioavailability in regard to the soy acids besides being an efficient energy source.

A very important clinical aspect in regard to the bioavailability is the slower metabolism in comparison to emulsions containing MCT, which are known to cause metabolic acidosis, when infused intravenously.

This invention is a selection invention in the sense that a general chemical formulation comprising the triglycerides according to the invention together with a very large number of other triglycerides belong to the prior art, vide EP 216 419, whereas the triglycerides according to the invention do not belong to the prior art. The triglycerides according to the invention are described and synthetized for the first time by the inventors, and also, the superior effect of the triglycerides according to the invention is demonstrated for the first time by the inventors.

The triglycerides according to the invention are 2-"soy"-1,3-didecanoyl glycerol. Surprisingly it has been found that these triglycerides exhibit a better absorption of the soy oil acids than the known soy oil acid sources in nutritional compositions.

An emulsion of the triglycerides 2-"Soy"-1,3-dioctanoyl glycerol is described in US 4,753,963. These triglycerides, however, are different from the triglycerides according to the invention, and also, they do not exhibit the useful and surprising properties described below for the products of the invention.

A preferred embodiment of the triglycerides according to the invention is characterized by the fact that they have a purity of at least 30%, preferably at least 50%, more preferably at least 75%, and most preferably at least 90%.

Surprisingly it has been found that these triglycerides exhibit a better bioavailability of soy acids than the known sources of soy acids when applied as nutrients e.g. formulated into an enterally administrable composition, and that the triglycerides of the invention exhibit better bioavailability than the known sources of soy acids, when formulated as a parenterally administrable composition. Also, it has been found that the enterally administrable composition according to the invention can be better tolerated and digested and exhibits a better smell and a more optimal nutrition than the previously known sources of soy acids. Thus, the enterally administrable composition according to the invention exhibits a vastly reduced tendency to diarrhoea, especially for infants. Moreover, the triglycerides of the invention turn out, surprisingly, to be very efficient sources of energy in enteral as well as in parenteral nutrition - the soy acid moiety of the triglycerides being in an optimal position in the molecule in respect to their cleavage by lipoprotein lipase. Also, the triglycerides of the invention appear to allow preservation of the soy acids in a hydrophobic form, in particular as compared to the corresponding octanoyl species, which can pass the intestinal mucosal layer and reach the enteroside in an optimal form for further esterification, lipoprotein transport and clearance.

Surprisingly, the triglycerides of the invention appear to exhibit physical properties which allow facile formulation of the compounds in liquid products as well as in powdered products exhibiting excellent wetability properties. The hydrophobicity of the products of the invention as compared to other sources of soy acids and in particular in comparison with the corresponding octanoyl species presumably is of importance for this behaviour and properties of the compounds of the invention. In the liquid form the products of the invention possess excellent stabilities making sterilization of e.g. parenteral products containing the triglycerides of the invention reliable, easy and safe.

The triglycerides of the invention allow formulation of feeding products containing non-deteriorated soy acids including the essential and polyunsaturated acids which are sensitive to oxidation and polymerisation processes. Detrimental processes of these polyunsaturated fatty acids leading to negative nutritional forms are thus commonly associated with gastric and intestinal problems due to oxidation and polymerisation products of the polyunsaturated fatty acid. Such oxidized forms of soy acids can interfere with nitrogen uptake by interacting with sensitive amino acids in infant formula. These highly undesired effects are diminished or even avoided by applying triglycerides according to the invention.

For use in parenteral feeding the triglycerides according to the invention can be incorporated into lipid emulsions where the liquid phase amounts up to 30% of the emulsion and they can be processed using the usual techniques to provide chylomicronlike particles. The products of the invention turn out to exhibit physical properties particularly advantageous in regard to this application as compared to the known sources of soy acids. The triglycerides of the invention can advantageously be applied in such emulsions due to their fast conversion by lipoprotein lipase and endothelial lipase and the consequential avoidance of the discomfort and side effects of lipolipaedemia. Soy acids applied in triglycerides of the invention are thus cleared quickly and efficiently thereby providing the essential fatty acid concomitantly with short chain acids useful as energy substrates.

The use of triglycerides according to the invention in parenteral nutritional products is further particularly advantageous since the relatively high polarity of the triglycerides favour the stability of their emulsions which are subjected to severe heat treatments during their manufacturing. Use of such emulsion is particularly advantageous for nutrition of pre-term babies, elderly individuals and diseased patients for whom use of the conventional triglyceride emulsions as e.g. those based on unmodified soy oil offer a range of disadvantages. For example, supply of fat emulsions to these individuals over extended periods of time of the conventional products leads to an over-supply of soy acids relative to the energy supply of the conventional products which do not adequately reach certain tissues as e.g. the small intestine. In contrast, the products of the invention contain in a single molecular type of species a more appropriate balance between the soy acids and the short chain fatty acids which are suitable for supply of energy. As compared to the octanoyl species the product of the invention possesses very useful physical properties in regard to their formulation into fat emulsions and they do provide a more adequate energy source for applications where the very high hydrophilicity of the octanoic acid is undesired.

A further advantage of the triglycerides of the invention is related to their solubility in other oils as e.g. vegetable oils. Such solutions can be used as such as a nutritional support. The triglycerides of the invention may also be formulated into creams and related products which allow the use of the compounds for topical treatment of e.g. skin diseases associated with essential fatty acid deficiencies.

Also the invention comprises a nutritional composition, which comprises the triglycerides according to the invention. This composition can be either the triglycerides according to the invention without the other constituents which are necessary for a full nutritional composition, i.e. mainly vitamins, proteins and carbohydrates, or the triglycerides according to the invention together with these other constituents.

A preferred embodiment of the nutritional composition is parenterally administrable. This composition is an emulsion.

A preferred embodiment of the nutrional composition is enterally administrable. This composition is either an emulsion or an oil.

A preferred embodiment of the enteral composition according to the invention is in fluid form. The enteral composition can be an emulsion or a pure oil.

A preferred embodiment of the enteral composition according to the invention is in powder form, whereby the triglycerides are encapsulated or microencapsulated. One of the manners, in which the droplets of triglyceride can be encapsulated, is described in Danochemo Technical Information on microencapsulated Product, Malmparken 5, 2750 Ballerup, Denmark.

Also the invention comprises a use of the nutritional composition according to the invention, as a nutrient or as part of a nutrient.

## Claims

1. The triglycerides 2-"soy"-1,3-didecanoyl glycerol.

2. Triglycerides according to Claim 1, which have a purity of at least 30%, preferably at least 50%, more preferably at least 75%, and most preferably at least 90%.

3. Nutritional composition, which comprises the triglycerides according to Claim 1 or 2.

4. Nutritional composition according to Claim 3, which is parenterally administrable.

5. Nutritional composition according to Claim 3, which is enterally administrable.

6. Nutritional composition according to Claim 5, which is in fluid form.

7. Nutritional composition according to Claim 5, which is in powder form, whereby the triglycerides are encapsulated or microencapsulated.

8. Use of the nutritional composition according to Claims 4 - 7, as a nutrient or as part of a nutrient.

## Patentansprüche

1. Die Triglyceride 2-"Soja"-1,3-didecanoylglycerin.

2. Triglyceride gemäß Anspruch 1, die eine Reinheit von wenigstens 30 %, vorzugsweise wenigstens 50 %, bevorzugter wenigstens 75 % und am bevorzugtesten wenigstens 90 % besitzen.

3. Nahrungszusammensetzung, welche die Triglyceride gemäß Anspruch 1 oder 2 umfaßt.

4. Nahrungszusammensetzung nach Anspruch 3, die parenteral verabreichbar ist.

5. Nahrungsmittelzusammensetzung nach Anspruch 3, die enteral verabreichbar ist.

6. Nahrungszusammensetzung nach Anspruch 5, die in flüssiger Form vorliegt.

7. Nahrungszusammensetzung nach Anspruch 5, die in Pulverform vorliegt, wobei die Triglyceride gekapselt oder mikrogekapselt sind.

8. Verwendung der Nahrungszusammensetzung nach den Ansprüchen 4 bis 7, als ein Nährmittel oder als Teil eines Nährmittels.

## Revendications

1. Triglycérides 2-"soja"-1,3-didécanoylglycérol.

2. Triglycérides selon la revendication 1, qui ont une pureté d'au moins 30 %, de préférence d'au moins 50 %, de façon plus préférable d'au moins 75 %, et de façon la plus préférable d'au moins 90 %.

3. Composition nutritive qui comprend des triglycérides selon la revendication 1 ou 2.

4. Composition nutritive selon la revendication 3, qui peut être administrée par voie parentérale.

5. Composition nutritive selon la revendication 3, qui peut être administrée par voie digestive.

6. Composition nutritive selon la revendication 5, qui est sous forme liquide.

7. Composition nutritive selon la revendication 5, qui est sous forme de poudre dans laquelle les triglycérides sont encapsulés ou microencapsulés.

8. Utilisation de la composition nutritive selon les revendications 4-7 comme aliment ou comme partie d'un aliment.
